# EUROPEAN PATENT SPECIFICATION

(11) **EP 2 736 903 B1**
(45) Date of publication and mention of the grant of the patent: **22.06.2016**
(21) Application number: 12820561.4
(22) Date of filing: 27.07.2012
(51) Int. Cl.: C07H 17/04, C07D 407/04, A61K 31/7048, A61P 31/18

(54) **ARYL NAPHTHALIDE LIGNANS AS ANTI-HIV AGENTS**
ARYL-NAPHTHALID-LIGNANE ALS ANTI-HIV-WIRKSTOFFE
LIGNANES DE NAPHTALIDE D'ARYLE EN TANT QU'AGENTS ANTI-VIH

(30) Priority: 29.07.2011 US 201161513119 P
(43) Date of publication of application: 04.06.2014
(73) Proprietor: The Board of Trustees of the University of Illinois, Urbana, IL 61801 (US)
(72) Inventor: ZHANG, Hongjie, Hong Kong (CN); SOEJARTO, Djaja, Lombard, Illinois 60148 (US); RONG, Lijun, Westmont, Illinois 60559 (US); FONG, Harry HS, Glen Ellyn, Illinois 60137 (US); RUMSCHLAG-BOOMS, Emily, Chicago, Illinois 60647 (US)
(74) Representative: Guardian IP Consulting I/S
(86) International application number: PCT/US2012/048657
(87) International publication number: WO 2013/019662

(56) References cited:
- US-A1- 2005 013 879
- US-A1- 2008 167 353
- A. S. R. ANJANEYULU ET AL: "New lignans from the heartwood of Cleistanthus collinus", TETRAHEDRON, vol. 37, no. 21, 1981, pages 3641-3652, XP002502258, ISSN: 0040-4020, DOI: 10.1016/S0040-4020(01)98893-3
- PATOOMRATANA TUCHINDA ET AL: "Cytotoxic arylnaphthalide lignan glycosides from the aerial parts of Phyllanthus taxodiifolius", PLANTA MEDICA, vol. 72, no. 1, 2006, pages 60-62, XP055154802, ISSN: 0032-0943, DOI: 10.1055/s-2005-87314
- JUNMIAN TIAN ET AL: "A new lignan and four new lignan glycosides from Mananthes patentiflora", HELVETICA CHIMICA ACTA, vol. 89, no. 2, 2006, pages 291-298, XP055154503, ISSN: 0018-019X, DOI: 10.1002/hlca.200690033
- CHIA-WEN CHANG ET AL: "Differential inhibition of reverse transcriptase and cellular DNA polymerase-[alpha] activities by lignans isolated from Chinese herbs, Phyllanthus myrtifolius Moon, and tannins from Lonicera japonica Thunb and Castanopsis hystrix", ANTIVIRAL RESEARCH, vol. 27, no. 4, 1995, pages 367-374, XP055154716, ISSN: 0166-3542, DOI: 10.1016/0166-3542(95)00020-M
- SHOEI-SHENG LEE ET AL: "Six lignans from Phyllanthus myrtifolius", JOURNAL OF NATURAL PRODUCTS, vol. 59, no. 11, 1996, pages 1061-1065, XP002732975, DOI: 10.1021/np960322+
- KADALI S. SAGAR ET AL: "Preparation and anti-HIV activities of retrojusticidin B analogs and azalignans", BIOORGANIC & MEDICINAL CHEMISTRY, vol. 12, no. 15, 2004, pages 4045-4054, XP055005478, ISSN: 0968-0896, DOI: 10.1016/j.bmc.2004.05.036
- PATOOMRATANA TUCHINDA ET AL: "Dichapetalin-type triterpenoids and lignans from the aerial parts of Phyllanthus acutissima", JOURNAL OF NATURAL PRODUCTS, vol. 71, no. 4, 2008, pages 655-663, XP055154519, ISSN: 0163-3864, DOI: 10.1021/np7007347
- ZHAO, Y. ET AL.: 'First synthesis of bioactive diphyllin glycosides isolated from justicia patentiflor hemsl' CHINESE JOURNAL OF CHEMISTRY vol. 25, 2007, pages 679 - 682, XP055143290
- SUSPLUGAS, S. ET AL.: 'Cytotoxic arylnaphthalene lignans from a vietnamese acanthaceae, Justicia patentiflora' JOURNAL OF NATURAL PRODUCTS vol. 68, 2005, pages 734 - 738, XP055143295

## Description

### BACKGROUND OF THE INVENTION

The present invention relates to anti-HIV agents originally derived from natural sources. More particularly, the agents are derivatives of aryl naphthalide lignans. It is a goal of the present invention to provide aryl naphthalide lignan compounds having anti-HIV activity.

AIDS has become a worldwide epidemic since its first report in 1981 in the US. In a UNAIDS (Uniting the world against AIDS) report, approximately 25 million people have died from AIDS (acquired immunodeficiency syndrome) and an estimated 33 million individuals are infected with the human immunodeficiency virus (HIV). AIDS is presently the leading cause of death in Africa and ranked as the fourth leading cause of death worldwide behind heart disease, stroke and respiratory infections. In Africa, it is estimated that more than 22 million people live with HIV and more than 2 million people are killed each year. According to the report by Mathers and Loncar (2006), AIDS is projected to be the third leading cause of death in 2030, and it is estimated that a total of nearly 120 million people could die from AIDS in the next 25 years (Mathers CD, Loncar D. PLoS Med 2006; 3: 2011-2030).

There are many anti-HIV therapeutic drugs currently in clinical use. The first anti-HIV drug, AZT (zidovudine), was developed by GlaxoSmithKline and approved in 1987. More anti-HIV drugs were introduced in the 1990s and 2000s. Today, more than 20 anti-HIV drugs are available on the market for HIV infected patients. Among these drugs, AZT, ddl (didanosine), ddC (zalcitabine), d4T (stavudine), 3TC (lamivudine), abacavir, tenofovir and emtricitabine were developed as nucleoside/nucleotide reverse transcriptase (RT) inhibitors; nevirapine, delavirdine, efavirenz and etravirine as nonnucleoside reverse transcriptase inhibitors; saquinavir, indinavir, ritonavir, nelfinavir, amprenavir, lopinavir/ritonavir, fosamprenavir, atazanavir, tipranavir and darunavir as protease inhibitors; enfuvirtide and maraviroc as entry and fusion inhibitors; and raltegravir as an integrase inhibitor. Although these drugs have significantly extended the life span of HIV-positive people in wealthy countries (Thayer AM. C&EN 2008; Sep 22: 29-36.), they are non-curative, and have the problems of side effects and diminishing effectiveness due to the development of viral resistance (Cos P, Maes L, Berghe DV, Hermans N, Pieters L, Vlietinck A. Journal of Natural Products 2004; 67: 284-293.). The fact that there currently are no drugs capable of curing, nor vaccine available to prevent this viral diseases, the discovery and development of new anti-HIV drugs are very much needed.

Aryl naphthalide lignans are compounds which comprise a structure of 2,3-dimethyl-1-phenyl-naphthalene. The compounds occur naturally and can also be synthesized. The structure of aryl naphthalide lignan itself is shown below:

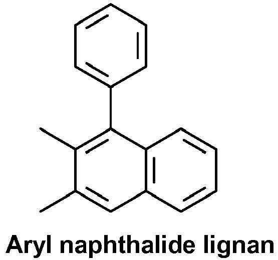

Some aryl naphthalide lignans have been reported to have anti-HIV activity in the literature (1. Chang CW, Lin MT, Lee SS, Liu KCSC, Hsu FL, Lin JY. Antiviral Research 1995; 27: 367-74. 2. Sagar KS, Chang CC, Wang WK, Lin JY, Lee SS. Bioorganic & Medicinal Chemistry 2004, 12:4045-4054. 3. Tuchinda P, Kornsakulkarn J, Pohmakotr M, Kongsaeree P, Prabpai S, Yoosook C, Kasisit J, Napaswad C, Sophasan S, Reutrakul V. Journal of Natural Products 2008; 71: 655-663.). However, none of the reported anti-HIV active aryl naphthalide lignans contain sugar units.

### SUMMARY OF THE INVENTION

The present invention is based, at least in part, on the discovery that glycosidic aryl naphthalide lignans compounds, such as justiprocumin A isolated from the plant *Justicia gendarussa Burm.f,* (Acanthaceae), are effective in the treatment of AIDS and HIV infections.

Accordingly, a first aspect of the invention is a compound selected from: 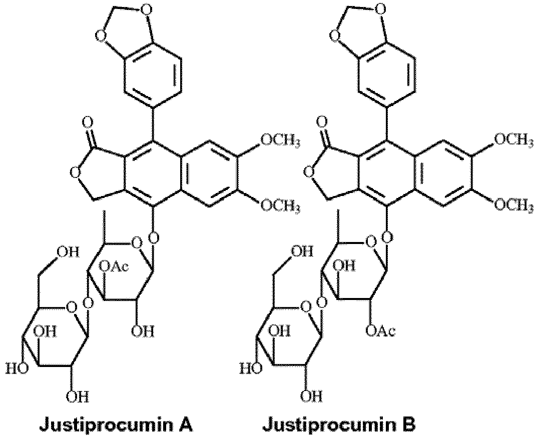 or a pharmaceutically acceptable salt thereof.

A second aspect of the invention is a compound for use in the treatment, prevention or delay of progression of a HIV infection in a patient, wherein the compound is selected from: 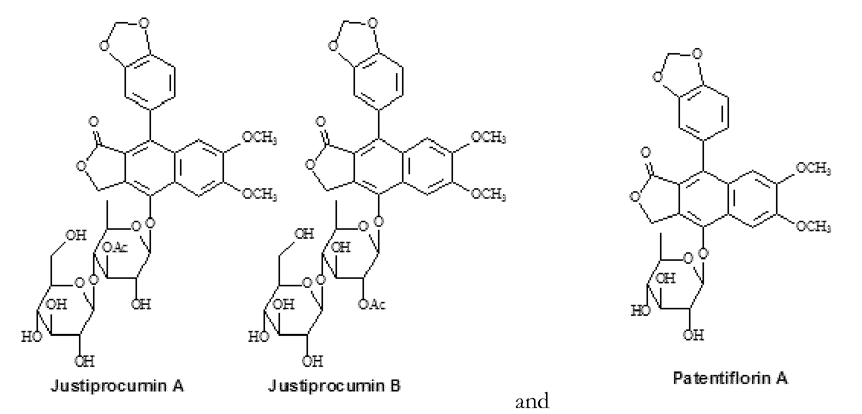 or a pharmaceutically acceptable salt thereof.

Compounds of the invention may exist in different forms, such as free acids, free bases, salts and tautomers.

### DETAILED DESCRIPTION OF THE INVENTION

### Plant Species and Anti-HIV Activity

*Justicia gendarussa* Burm.f. belongs to Acanthaceae family. It is a bush up to about 0.3 m in height, and is found in Vietnam, China, Cambodia, India, Indonesia, Laos, Malaysia, Myanmar, Papua New Guinea, Philippines, Sri Lanka, Thailand (Flora of China). The fresh leaves have been traditionally used in these countries for treatment of muscle pains, broken/fractured bone, muscle sprains, cuts, hemiplegia, rheumatism, arthritis, headache and earache. The methanol extract of the whole plant of this plant was determined by us to be highly potent against HIV replication. The methanol extract made from the stem barks of this plant demonstrated capability to inhibit 50 % HIV replication at a concentration of 0.04 µg/mL. Further phytochemical study of this plant was thus conducted to identify the anti-HIV active compounds contained in this plant.

### Bioassay Directed-Fractionation

Bioassay directed-fractionation of the stem barks of *J. gendarussa* led to the isolation of the new aryl naphthalide lignan compounds of the present invention. "Bioassay-directed fractionation" is the sequential reduction of complex mixtures eventually to individual components. The extracts are tested for biological effects and subjected to one or several fractionation procedures. After each separation step the fractions are evaluated for biological activity for selection of active fractions for further investigation. When the complexity of the mixture is reduced to a few individual compounds, the fractions will be purified to obtain bioactive compounds, which are subjected to chemical identification and further bioactivity evaluation.

### Glycoside/Glycosidic Compound

The terms "glycoside" or "glycosidic compound" as used herein are interchangeable and includes reference to any of the class of compounds that yield a sugar and an aglycone upon hydrolysis.

### Aryl Naphthalide Lignan

The term "aryl naphthalide lignan" as used herein includes reference to a compound comprising the basic structure of 2, 3-dimethyl-1-phenyl-naphthalene shown as below:

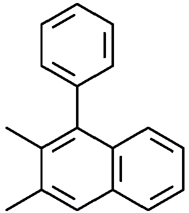

In one class of aryl naphthalide lignan compounds, the two methyl groups are forming a dihydro-furan-2-one ring, while in another class the two methyl groups are forming a dihydro-furan-3-one ring. Both classes of compounds have been found to be natural products.

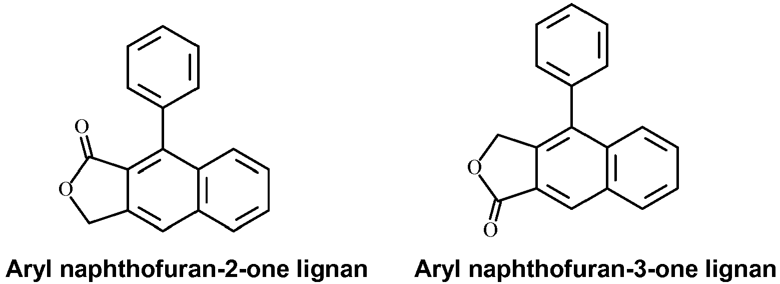

### Compounds

The invention involves the aryl naphthalide lignan compounds of patentiflorin A, justiprocumin A or justiprocumin B, or pharmaceutically acceptable salts thereof.

The compounds of the invention are shown below. It will of course be appreciated that, where appropriate, each compound may be in the form of the free compound, an acid or base addition salt.

### Examples

The root plus stem sample of *Justicia gendarussa* Burm.f. (Acanthaceae) was collected from Cue Phuong National Park (Nho Quan District, Ninh Binh Province, Vietnam). Its methanol extract exhibited potent inhibition activity against HIV replication with an IC50 value of 0.04 µg/mL. A 4.0 kg sample of the dried root plus stem of this plant was thus re-collected from the Cue Phuong National Park in Vietnam in order to identify anti-HIV compounds. As a result, two new (1 and 2) and one known (3) aryl naphthaiide lignans were isolated from this plant by bioassay-guided fractionation studies. In the structures of 1 and 2, Ac represents as an aceyl (-COCH3) group, and Glu represents as a β-D-glucopyranosyl group.

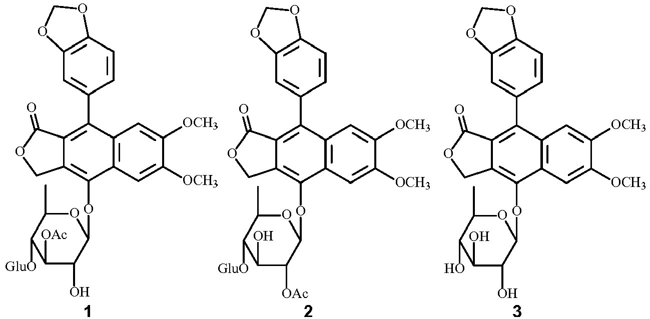

The recollected stem bark (4.0 kg) of *J. procumbens* was extracted with MeOH to afford an extract (155 g). Bioassy-directed fractionation of the MeOH extract by column chromatography on Si gel and RP-18 Si gel and subsequent preparative HPLC separation led to the isolation of two new aryl naphthalide lignans, justiprocumin A (**1**) and justiprocumin B (**2**), together with a known aryl naphthalide lignan, patentiflorin A (**3**).

Justiprocumin A (**1**) was obtained as a white powder with a molecular formula of C₃₅H₃₈O₁₇ by positive HRESIMS ([M+H]⁺*m*/*z* 731.2217, calcd. 731.2187) and NMR studies (Tables 1 and 2). The compound was rapidly elucidated to be an aryl naphthalide lignan by comparison of its ¹H and ¹³C NMR data to those of aryl naphthalide lignan compounds. In combination of analysis 2D NMR data including ¹H-¹H COSY, HMQC and HMBC spectral data, **1** was determined as 9-(1, 3-benzodioxol-5-yl)-4-[(3-O-acetyl-6-deoxy-4-O-β-D-glucopyranosyl-β-D-glucopyranosyl)oxy]-6, 7-dimethoxy- naphtho[2,3-c]furan-1(3*H*)-one, and was given the trivial name justiprocumin A.

Justiprocumin B (**2**) was obtained as a white powder with the same molecular formula (C₃₅H₃₈O₁₇) to that of **1**, which was determined by positive HRESIMS ([M+H]⁺ *m*/*z* 731.2216, calcd. 731.2187) and NMR studies (Tables 1 and 2). The structure of this compound is very similar to that of **1**. Compound **2** differs from **1** only by the position of an acetyl group. In combination with analysis of 2D NMR data including ¹H-¹H COSY, HMQC and HMBC spectral data, **2** was determined as 9-(1, 3-benzodioxol-5-yl)-4-[(2-O-acetyl-6-deoxy-4-O-β-D-glucopyranosyl-β-D-glucopyranosyl)oxy]-6,7-dimethoxy-naphtho[2, 3-c]furan-1 (3*H*)-one, and was given the trivial name justiprocumin B. 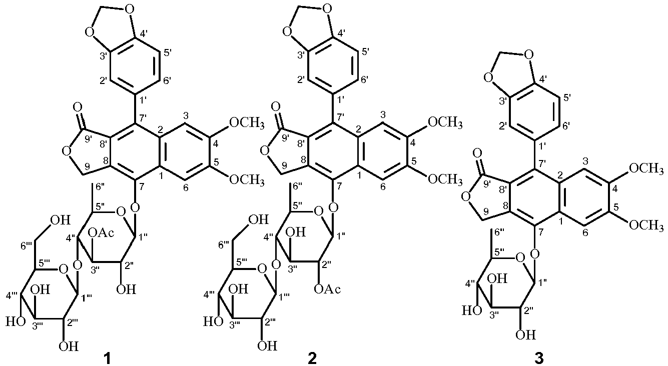 Compound **3** was obtained as the known compound patentiflorin A (Susplugas S, Nguyen VH, Bignon J, Thoison O, Kruczynski A, Sevenet T, Gueritte F. Journal of Natural Products 2005, 68: 734-738.) by analysis of its spectroscopic data (Tables 1 and 2), and a comparison of the data with those reported in literature. We further totally synthesized patentiflorin A. The synthesized patentiflorin A showed the same ¹H and ¹³C NMR spectral data as those of the natural patentiflorin A, which confirmed the chemical structure of patentiflorin A (Scheme 1).

**Table 1. ¹H NMR Spectral Data of Compounds 1-3 (1 and 2: 500 MHz; 3: 360 MHz; CD₃OD; J in Hz)**

| | **1** | **2** | **3** |
|---|---|---|---|
| Position | | *δ* (ppm) | |
| H-3*^{a}* | 7.011 s | 6.962 s | 6.971 s |
| | 6.992 s | 6.954 s | 6.951 s |
| H-6*^{a}* | 8.052 s | 7.496 s | 8.052 s |
| | 8.043 s | 7.493 s | 8.042 s |
| H-9a*^{a}* | 5.550 d (15.2) | 5.471 brs | 5.537 d (15.2) |
| | 5.546 d (15.2) | | 5.531 d (15.2) |
| H-9b*^{a}* | 5.418 d (15.1) | 5.471 brs | 5.410 d (15.2) |
| | 5.415 d (15.2) | | 5.406 d (15.4) |
| H-2'*^{a}* | 6.766 d (1.8) | 6.712 d (1.6) | 6.730 d (1.6) |
| | 6.725 overlap | 6.657 d (1.6) | 6.655 d (1.7) |
| H-5'*^{a}* | 6.929 d (7.8) | 6.908 d (7.9) | 6.897 d (7.9) |
| | 6.919 d (7.7) | | |
| H-6'*^{a}* | 6.723 dd (7.9, 1.9) | 6.685 dd (7.9, 1.7) | 6.682 dd (7.7, 1.8) |
| | 6.715 dd (7.7, 1.8) | 6.665 dd (7.8, 1.9) | 6.659 dd (7.8, 1.7) |
| H-1"*^{a}* | 4.852 d (7.9)*^{f}* | 5.184 d (8.1) | 4.761 d (7.8) |
| | 4.842 d (7.9)*^{f}* | 5.181 d (8.0) | 4.760 d (7.9) |
| H-2"*^{a}* | 3.862 dd (9.2, 7.7) | 5.266 dd (9.7, 7.9) | 3.638 dd (9.5, 8.1) |
| | 3.858 dd (9.4, 8.0) | 5.248 dd (9.8, 7.9) | 3.634 dd (9.1, 8.0) |
| H-3" | 5.035 t (9.0) | 3.756 brt (9.0) | 3.408 t (9.1) |
| H-4" | 3.603 t (9.4) | 3.453 t (9.1) | 3.316 t (9.5) |
| H-5" | 3.522 dq (9.3, 5.9) | 3.668 m | 3.266 m |
| CH₃-6"*^{a}* | 1.425 d (6.0) | 1.465 d (6.2) | 1.303 d (6.0) |
| | 1.422 d (6.1) | 1.464 d (6.1) | 1.299 d (6.1) |
| H-1"' | 4.319 d (7.7) | 4.431 d (7.8) | |
| H-2"' | 3.100 dd (9.2, 8.0) | 3.229 dd (9.3, 8.0) | |
| H-3"' | 3.325 t (8.8) | 3.377 t (9.0) | |
| H-4"' | 3.220 dd (9.5, 8.7) | 3.272 brt (9.4) | |
| H-5"' | 3.285 ddd (8.6, 6.2, 2.4) | 3.371 m | |
| H-6"'a | 3.918 dd (11.6, 2.1) | 3.914 dd (11.9, 2.1) | |
| H-6"'b | 3.667 dd (11.6, 6.2) | 3.645 dd (11.7, 6.1) | |
| 3'-OCH₂O- | 6.045 m | 6.036 m | 6.031 m |
| 4'*^{a}* | 6.029 m | 6.018 m | 6.019 m |
| 4-OCH₃*^{a}* | 3.683 s | 3.679 s | 3.670 s |
| | 3.675 s | 3.676 s | 3.664 s |
| 5-OCH₃*^{a}* | 3.961 s | 3.975 s | 3.974 s |
| | 3.959 s | 3.972 s | 3.970 s |
| 2"- | | 2.113s | |
| OCOCH₃*^{a}* | | 2.111 s | |
| 3"-OCOCH₃ | 2.185 s | | |

| | | | |
|---|---|---|---|
| ^{a} The ¹H NMR signals were shown to be doubling due to some degree of hindered rotation of the aryl-naphthalene bond. | | | |

**Table 2. ¹³C NMR and DEPT Spectral Data of Compounds 1-3 (1 and 2: 500 MHz; 3: 360 MHz; CD₃OD)**

| | **1** | **2** | **3** |
|---|---|---|---|
| Position | *δ* (ppm) | | |
| C-1 | 128.87 s | 127.36 s | 128.84 s |
| C-2 | 131.88 s | 131.60 s | 131.79 s |
| C-3 | 106.93 d | 106.91 d | 106.81 d |
| C-4 | 151.71 s | 151.77 s | 151.62 s |
| C-5 | 153.37 s | 153.25 s | 153.23 s |
| C-6 | 102.62 d | 101.86 d | 102.66 d |
| C-7 | 146.24 s | 145.96 s | 146.34 s |
| C-8*^{a}* | 132.18 s | 127.57 s | 132.22 s |
| | | 127.50 s | |
| C-9 | 69.05 t | 68.63 t | 69.10 t |
| C-1' | 129.89 s | 129.85 s | 129.89 s |
| C-2' | 111.75 d | 111.75 d | 111.75 d |
| C-3' | 148.99 s | 148.93 s | 148.94 s |
| C-4' | 148.99 s | 148.93 s | 148.94 s |
| C-5' | 108.97 d | 108.94 d | |
| C-6'*^{a}* | 124.73 d | 124.74 d | 124.75 d |
| | | | 124.71 d |
| C-7' | 137.74 s | 136.75 s | 137.50 s |
| C-8'*^{a}* | 119.96 s | 120.14 s | 119.89 s |
| | 119.92 s | | 119.84 s |
| C-9' | 172.08 s | 171.76 s | 172.16 s |
| C-1" | 106.35 d | 101.86 d | 106.68 d |
| C-2" | 73.73 d | 75.18 d | 75.79 d |
| C-3" | 76.79 d | 74.12 d | 77.80 d |
| C-4" | 81.93 d | 85.77 d | 76.69 d |
| C-5" | 73.03 d | 72.91 d | 73.63 d |
| C-6" | 18.13 q | 18.07 q | 18.20 q |
| C-1"' | 104.72 d | 105.01 d | |
| C-2"' | 75.31 d | 75.07 d | |
| C-3"' | 77.93 d | 77.93 d | |
| C-4"' | 71.89 d | 71.51 d | |
| C-5"' | 78.04 d | 78.22 d | |
| C-6"' | 63.04 t | 62.59 t | |
| 3'-OCH₂O-4' | 102.62 t | 102.9 t | 102.61 d |
| 4-OCH₃ | 55.99 q | 56.01 q | 55.97 q |
| 5-OCH₃ | 56.69 q | 56.68 q | 56.69 q |
| 2"-OCOCH₃ | | 21.30 q | |
| 3"-OCOCH₃ | 21.46 q | | |
| 2"-OCOCH₃ | | 171.76 s | |
| 3"-OCOCH₃ | 173.11 s | | |

| | | | |
|---|---|---|---|
| *^{a}*The ¹³C NMR signals were shown to be doubling due to some degree of hindered rotation of the aryl-naphthalene bond. | | | |

Our **"Anti-HIV Post-Entry"** protocol has been used for initial evaluation of anti-HIV activity of fractions and isolated compounds. By using this protocol, all three isolated compounds (**1-3**) exhibited potent inhibition of HIV replication. Patentiflorin A (**3**) demonstrated anti-HIV activity with an IC₅₀ value of less than 10 ng/mL, and both justiprocumins A (**1**) and B (**2**) inhibited HIV replication by more than 100 % at a concentration of 1 µg/mL.

Justiprocumins B (**2**) and patentiflorin A (**3**) were further evaluated against a broad spectrum of HIV strains including four HIV1 clinical strains, BAL and SF162 (M-tropic), BAL (T-tropic), and 89.6 (a dual tropic strain). In these experiments, AZT was used as a positive control. Justiprocumin A (**1**) was not tested since it is structurally similar to justiprocumin B (**2**) and exhibited a similar inhibition profile to that of Justiprocumin B (**2**) using the "One-Stone-Two Birds" protocol. It is clear that justiprocumin B (**2**) and patentification A (**3**), like AZT, could inhibit the particle production of all four HIV-1 strains effectively in a dose-dependent manner. Justiprocumin B (**2**) gave an IC₅₀ value of 14-21 nM, and patentification A (**3**) an IC₅₀ value of 24-37 nM, compared to 77-95 nM for AZT, depending on the strains (Table 3).

**Table 3. Anti-HIV activity of justiprocumin B (2) and patentiflorin A (3) against four HIV strains.**

| | IC₅₀ (nM) | | | |
|---|---|---|---|---|
| | Bal: M-Tropic | 89.6: Dual-Tropic | SF162: M-Tropic | Lav.04: T-Tropic |
| Justiprocumin B (**2**) | 15 | 15 | 14 | 21 |
| Natural patentiflorin A (**3**) | 30 | 32 | 37 | 24 |
| Synthetic patentiflorin A (**3**) | 32 | 31 | 30 | 32 |
| AZT | 77 | 95 | 85 | 79 |

As shown above, these identified potent anti-HIV compounds are structurally similar. To determine their toxicity, patentiflorin A was chosen to be evaluated for its toxicity against the A549 and Hela cell lines. At a concentration of 10 µg/mL, patentiflorin A exhibited no apparent cytotoxicity against A549 and Hela cell lines.

To confirm the anti-HIV activity of the aryl naphthalide lignan glycosides, we synthesized, de novo, one (patentiflorin A) of the aryl naphthalide lignan glycosides. Patentiflorin A was isolated as a potent anti-HIV aryl naphthalide lignan glycoside compound from the stems and roots-of *Justicia procumbens* through bioassay-guided fractionation. In order to confirm its anti-HIV activity and obtain a sufficient amount of this compound for further biological study, we undertook to totally synthesize this compound, de novo. Scheme 1 showed our successful route in synthesizing patentiflorin A. The spectral data (MS and NMR) of the synthetic compound being the same as those of the natural isolate confirmed that the two compounds are of the same structure. We further determined that the synthetic patentiflorin A (SF85) showed similar anti-HIV activity as that of the isolated patentiflorin A. We further evaluated inhibition of natural patentiflorin A and synthetic patentiflorin A against the four HIV-1 strains (**Table 3**). Bioassay showed that the synthetic patentiflorin A not only retained the anti-HIV potency in comparison of the natural patentiflorin A, but also had a better therapeutic index in comparison of the natural compound (SI > 2,500). We further investigated this compound against HIV gene expression of the R/U5 and U5/gag transcripts. Our data showed that the compound acts as an effective inhibitor of HIV reverse transcription. Thus, we have confirmed the anti-HIV activity of the aryl naphthalide lignan compounds through chemical synthesis and biological approach.

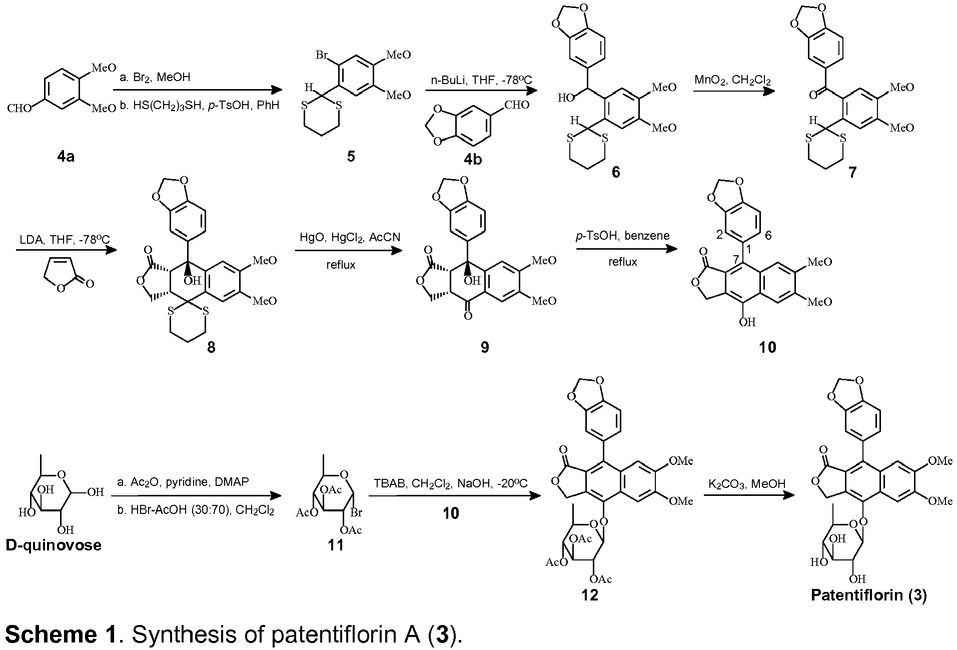

*General Experimental Procedures.* Optical rotations were measured on a Perkin-Elmer model 241 polarimeter. IR spectra were run on a Jasco FT/IR-410 spectrometer, equipped with a Specac Silver Gate ATR system by applying a film on a Germanium plate. 1 D and 2D NMR spectra were recorded on a Bruker Avance-360 or Avance-500 MHz spectrometer. Column chromatography was carried out on silica gel (200-400 mesh, Natland International Corporation), and reversed-phase flash chromatography was accomplished with RP-18 silica gel (40-63 µ, EM Science). Reversed-phase HPLC was carried out on a Waters 600E Delivery System pump, equipped with a Waters 996 photodiode detector, and a Phenomenex LUNA C18 column (10 µ, 250 × 50 mm), which also resulted in extracting UV spectral data of each purified compound. Thin-layer chromatography was performed on Whatman glass-backed plates coated with 0.25 mm layers of Silica gel 60. HRTOFMS spectra were recorded on a ThermoFinnigan LTQFT spectrometer.

*Plant Material.* The initial collection of root and stem sample (SV5614) of *Justicia procumbens* L. (Acanthaceae) was made in Phu Lai at Hoa Binh province side of Cuc Phuong National Park, with geographic coordinate readings of 20° 21' 633" N 105° 38' 257" E, and voucher herbarium specimen Mai Van Xinh 742. A larger amount of the plant sample for the current isolation work, consisting of the same plant part (bark and stem), sample SVA5614 (4.0 kg), was subsequently re-collected from plants located in the same area. The recollection of root and stem sample (SVA5614) of J. gendarussa was made near Cave of Prehistoric Man close to hotspot 33 of Cue Phuong National Park, with geographic coordinate readings of 20° 17' 516" N, 105° 40' 211 " E. Voucher specimens (*N.M. Cuong* #*2162*) have been deposited at the Herbarium of Cuc Phuong National Park.

*Extraction and Isolation.* The dried, milled plant material (4.0 kg) was extracted with methanol to afford 155 g of extract. A small portion of the methanol extract (5.5 g) was rapidly fractionated to 22 fractions (Fa1-Fa22) over a silica gel (10 g) column eluting with gradient CHCl₃, CHCl₃/Me₂CO, and CHCl₃/MeOH solutions. Anti-HIV evaluation of the fractions determined that the fractions Fa14-Fa18 inhibited HIV replication by more than 95 % at a concentration of 0.1 µg/mL without apparent toxicity at 20 µg/mL. HPLC separation of Fa16 led to the isolation of justiprocumins A (**1**) and B (**2**). Scale-up fractionation of SVA5614 was then carried out in attempt to isolate other anti-HIV active compounds in this plant. The methanol extract (140 g) was absorbed in 214 g silica gel, and chromatographed over a silica gel column (1545 g). The column was developed by gradient elution with CHCl₃, CHCl₃/Me₂CO, and CHCl₃/Me₂CO/MeOH solutions to afford 40 fractions. Fraction F26 showed very similar TLC and HPLC profiles to those of Fa16. A portion of F26 was thus subjected preparative HPLC separation to yield 8 fractions (F41-F48). Bioassay showed that the fraction F47 was able to inhibit HIV replication by more than 90 % at a concentration of 0.2 µg/mL. HPLC separation of F47 led to the isolation of patentiflorin A (**3**).
*Justiprocumins A (**1**)*. White powder, [α]20,D -26.2° (c 0.55, MeOH); UV λmax [AU (absorbance units)] = 201.6 (0.77), 225.2 (0.41), 261.5 (1.01), 294.0 (0.19), 314.9 (0.21), 356.1 (0.07) nm; IR (film) vₘₐₓ 3421 (*br*)*,* 2924, 1745, 1622, 1507, 1480, 1456, 1435, 1391, 1341, 1262, 1229, 1168, 1066, 1034, 930, 865, 770 cm⁻¹; ¹H and ¹³C NMR data, see Tables 1 and 2; positive HRESIMS [M+H]⁺*m*/*z* 731.2217 [M+H]⁺ (calcd. for C₃₅H₃₈O₁₇, 731.2187).
*Justiprocumins B* (***2***)*.* White powder, [α]_{20,D} -32.0° (c 0.74, MeOH); UV λmax [AU (absorbance units)] = 201.6 (1.16), 226.5 (0.63), 261.5 (1.41), 293.6 (0.29), 314.9 (0.31), 356.0 (0.14) nm; IR (film) vₘₐₓ 3422 (*br*), 2924, 1748, 1651, 1621, 1558, 1541, 1507, 1476, 1456, 1435, 1450, 1262, 1227, 1168, 1068, 1035, 931, 863, 770 cm⁻¹; ¹H and ¹³C NMR data, see Tables 1 and 2; positive HRESIMS [M+H]⁺ *m*/*z* 731.2216 [M+H]⁺ (calcd. for C₃₅H₃₈O₁₇, 731.2187).
*Patentiflorin A* (***3***). White powder, [α]_{20,D} -20.4° (c 1.61, MeOH); UV λmax [AU (absorbance units)] = 200.4 (1.06), 225.1 (0.54), 261.5 (1.29), 293.9 (0.24), 314.9 (0.26), 355.0 (0.10) nm; ¹H and ¹³C NMR data, see Tables 1 and 2.

Total Synthesis of Patentiflorin A (**3**) (Scheme 1). To a solution of veratral (**4a**) (63.2 mmol) in methanol (MeOH, 250 mL), a solution of bromine (Br₂, 69.5 mmol) was added slowly. The reaction was allowed to stir at room temperature for 6 hr to afford 2-bromo-veratral (13.4 g, 86 %). 2-Bromo-veratral (50.3 mmol) was then dissolved in 300 mL benzene, to which, 1, 3-propanedithiol (50.4 mmol) and p-toluenesulfonic acid (p-TsOH, 2.5 mmol) were added. The reaction mixture was allowed to be stirred at reflux for 10 hr then ambient temperature for 48 hr to yield **5** (15.6 g, 93 %). To a stirred solution of compound **5** (46.6 mmol) in tetrahydrofuran (THF, 150 mL) at -78 °C was added n-butyllithium (n-BuLi) (1.6 M solution in hexanes, 69.9 mmol) in 1 hr, followed by addition of a solution of piperonal (**4b**) (55.9 mmol) in THF (30 mL). The reaction was allowed for a further 2 hr and then 3 hr at room temperature to yield **6** (9.5 g, 50 %). Compound **6** (20.7 mmol) in CH₂Cl₂ (200 mL) was added a suspension of activated manganese dioxide (MnO₂, 345 mmol) at room temperature. The reaction was allowed for 16 hr, and was then filtered through a plug of celite to afford the oxidative product **7** (8.2 g, 98 %). n-Butyllithium (1.6M solution in hexanes, 100 mmol) was added dropwise over 5 min to a cooled (-78 °C) solution of diisopropylamine (100 mmol) in THF (43 mL) under Argon to make fresh lithium diisopropylamide (LDA) solution. The solution was warmed to ambient temperature over 30 min and 25.7 mL added, dropwise via syringe over 3 min to a cooled (-78 °C) THF solution (60 mL) of the dithiane **7** (14.29 mmol). After 40 min, 2,5-dihydrofuranone (17.1 mmol) as a solution in THF (10 mL) was added over 1 min. The reaction is warmed to ambient temperature for 1 hr to afford **8** (3.95 g, 57 %). A solution of compound **8** (0.65 mmol), mercury oxide (HgO, 0.71 mmol) and mercury chloride (HgCl₂, 1.43 mmol) in 84 % acetonitrile (AcCN) aqueous (25 mL) was heated to reflux for 3 hr to afford **9** (134 mg, 52 %). The ketone **9** (0.34 mmol) and p-TsOH (0.19 mmol) were heated to reflux in benzene (150 mL) for 16 hr to yield the desired compound diphyllin (**10**, 106 mg, 83 %). To the solution of D-quinovose (0.61 mmol) in pyridine (5 mL) was added 4-dimethylaminopyridine (DMAP, 0.06 mmol) and acetic anhydride (Ac₂O, 1.5 mL) at room temperature, and the mixture was stirred overnight and quenched with MeOH (1 mL) to afford 1,2,3,4-tetraacetyl-D-quinovose. Without further separation, the tetraacetate was dissolved in glacial acetic acid (AcOH, 1 mL), and 1.5 mL hydrobromic acid (HBr) (33% in AcOH) was added to the solution slowly at room temperature. The reaction was allowed for 15 min to afford **11** (215 mg, 97 %). To a solution of TBAB (tetrabutylammonium bromide, 0.95 mmol) and diphyllin (**10**, 0.95 mmol) in 15 mL of dichloromethane (CH₂Cl₂) was added aqueous 0.1 M sodium hydroxide (NaOH, 20 mL). After stirring for 10 min at 40 °C, compound **11** (0.61 mmol) was then added, and the two-phase reaction was stirred for 6 hr at 40 °C to afford the solid **12**. Without further separation, the solid **12** (538 mg) was dissolved in MeOH (10 mL), and potassium carbonate (K₂CO₃, 1.0 mmol) was then added. The reaction was allowed for 1 hr to afford patentiflorin A (**3**) (122 mg).

*Anti-HIV Evaluation Using Post-Entry Protocol.* This protocol allows us to identify potential inhibitors for HIV replication (post-entry steps). In this system, the HIV vector pNL4-3.Luc. R.E (Connor RI, Chen BK, Choe S, Landau NR. Virology 1995; 206: 935-44; and He J, Choe S, Walker R, Di Marzio P, Morgan PO, Landau NR. Journal of Virology 1995; 69: 6705-11.), obtained through the AIDS Research and Reference Reagent Program (Division of AIDS, NIAID, NIH), was co-transfected with the H5N1 HA and NA constructs to generate HIV virions with bird flu HA on the viral surface (HIV/HA). This pNL4-3 was derived from an infectious molecular clone of an SI, T-tropic virus (Michael NL, Nelson JA, KewalRamani VN, Chang G, O'Brien SJ, Mascola JR, Volsky B, Louder M, White GC 2nd, Littman DR, Swanstrom R, O'Brien TR. Journal of Virology 1998; 72: 6040-7.), and is replication deficient since the HIV is Env⁻ and Vpr⁻. Also the luciferase gene (luc) carried by this recombinant HIV vector serves as the reporter for HIV replication (reverse transcription, integration and HIV gene expression). The evaluation principle is that the level of the luciferase activity in the cells should be proportional to the level of viral entry and replication. If a compound (or fraction) can interfere with HIV replication/or HA-mediated viral entry, the level of the luciferase activity in the infected cells will be reduced. Thus, using this protocol, we were able to identify fractions or compounds capable of inhibiting HIV replication. The fractions or compounds were evaluated as follows. The stock HIV/HA virions (approximately 2 × 10⁶ relative light units, or RLUs, on the target cells) were mixed with the individual extract first, and the mixture was incubated with the target cells in 24 well plates (human lung cell line A549 was used since it is susceptible to HA-mediated viral entry). The final concentration of the extract was 20 µg/ml. Forty-eight hours post-infection, the target cells were lysed and the luciferase activity was determined.

*Anti-HIV Evaluation Using Four HIV1 Clincal Strains.* Four HIV1 clinical strains, BAL and SF162 (M-tropic), BAL (T-tropic), and 89.6 (a dual tropic strain), were used in the current study. Here a standardized human peripheral blood mononuclear cell culture (PBMC) assay was used to determine the compound susceptibility of these HIV-1 strains. AZT, an anti-HIV drug in clinical use, was used as a positive control. Briefly, human PBMCs were collected from a donor and stimulated for seven days. The preparations (compound or fraction) were then added to the cultured cells at a wide range of concentrations, and the different HIV-1 strains were used to challenge the cultured cells using 96-well plates. After seven-days of incubation, the supernatants were collected and the HIV p24 levels of the infected cells were determined using a p24 antigen ELISA. The IC₅₀s were calculated by comparing p24 antigen values for the compounds (fraction)-containing wells with those for no drug control wells. In these experiments, AZT was used as positive controls.

*Evaluation of Toxicity.* Approximately 5000 cells seeded in a 96-well tissue culture plate in DMEM supplemented with FBS and penicillin/streptomycin. DMSO alone or compounds in 100% DMSO were added on the following day at appropriate concentrations and incubated with cells at 37 °C in 5% CO₂ for 24 hr. After 24 hr, all media were removed and replaced with 100 µL fresh complete DMEM. After 48 hr post initial addition of DMSO or compound, 20 µL of CellTiter 96 Aqueous One Solution was added per well. After gentle mixing, plates were incubated at 37 °C in 5% CO₂ for 4 hr. 25 µL of a 10% SDS solution was added per well and plates were stored at room temperature for approximately 12 hr. Absorbance was then measured at 450nm using a plate reader.

## Claims

1. A compound selected from: 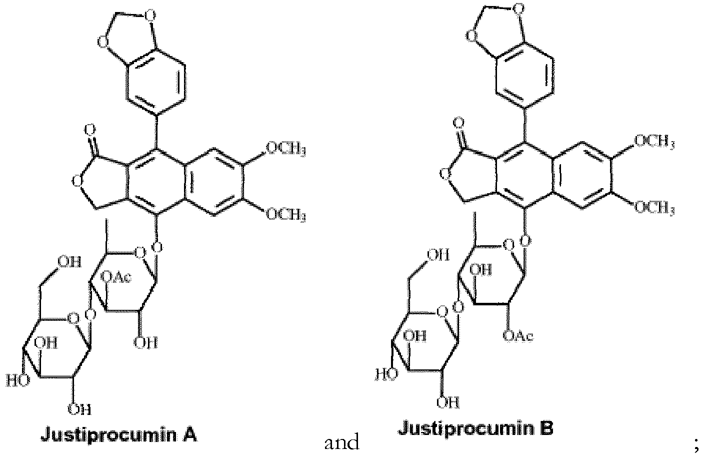 or a pharmaceutically acceptable salt thereof.

2. A compound for use in the treatment, prevention or delay of progression of a HIV infection in a patient, wherein the compound is selected from: 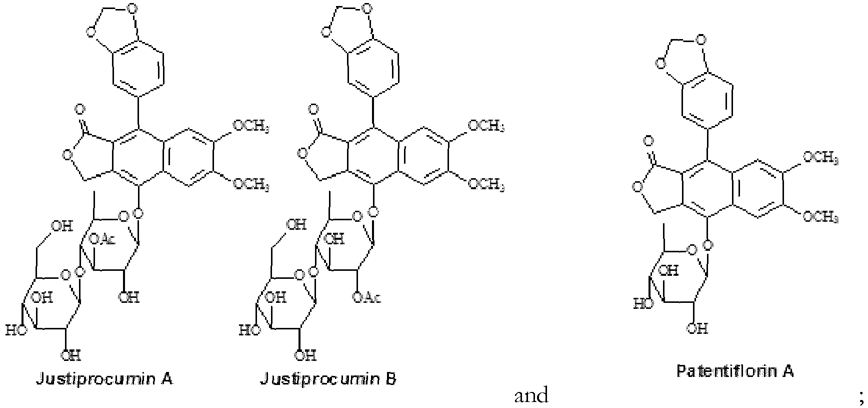 or a pharmaceutically acceptable salt thereof.

3. The compound for use according to claim 2, wherein the compound is 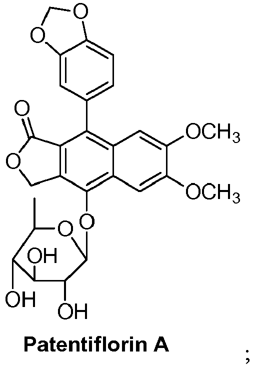 or a pharmaceutically acceptable salt thereof.

## Patentansprüche

1. Verbindung ausgewählt von: 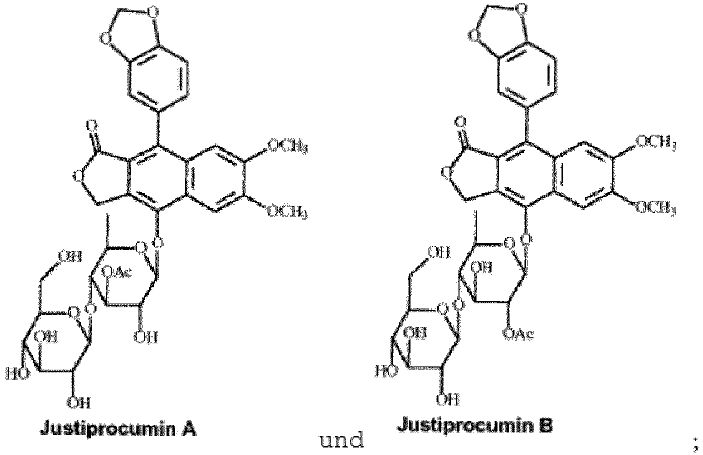 oder einem pharmazeutisch akzeptablen Salz davon.

2. Verbindung zur Verwendung bei der Behandlung, Prävention oder Verzögerung des Fortschreitens einer HIV-Infektion bei einem Patienten, wobei die Verbindung ausgewählt wird von: 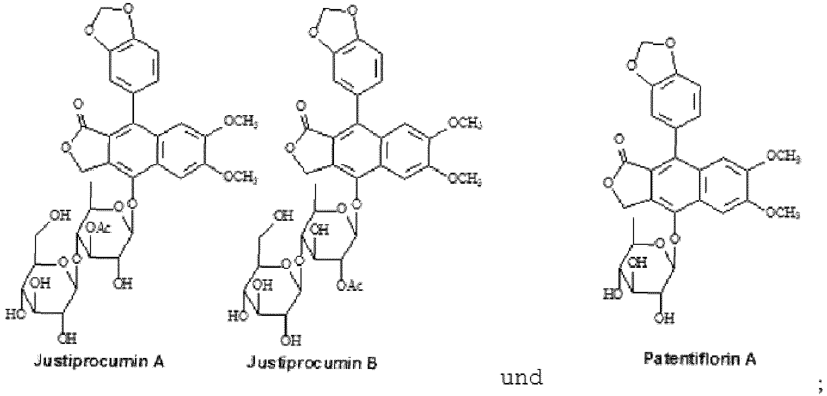 oder einem pharmazeutisch akzeptables Salz davon.

3. Verbindung zur Verwendung nach Anspruch 2, wobei die Verbindung 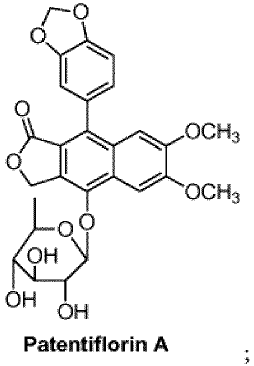 oder ein pharmazeutisch akzeptables Salz davon ist.

## Revendications

1. Composé sélectionné à partir de : 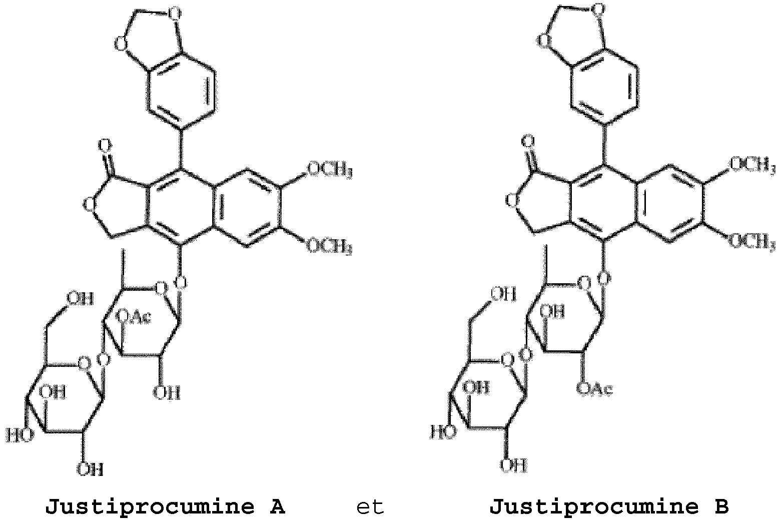 ou un sel de ce dernier acceptable d'un point de vue pharmaceutique.

2. Composé à utiliser dans le traitement, la prévention ou le retard de la progression d'une infection par VIH chez un patient, dans lequel le composé est sélectionné à partir de : 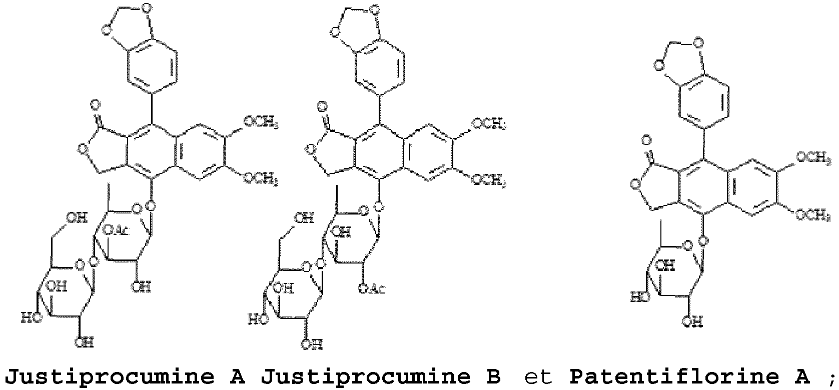 ou un sel de ce dernier acceptable d'un point de vue pharmaceutique.

3. Composé à utiliser selon la revendication 2, dans lequel le composé est 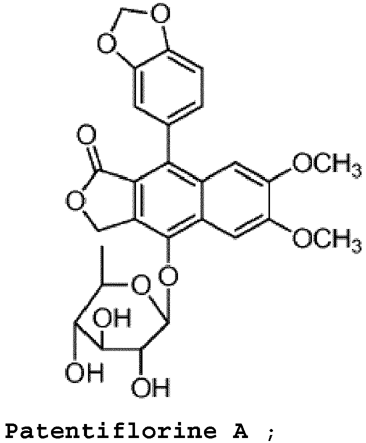 ou un sel de ce dernier acceptable d'un point de vue pharmaceutique.
